# FASCICULE DE BREVET EUROPEEN

(11) **EP 2 654 752 B1**
(45) Date de publication et mention de la délivrance du brevet: **27.05.2015**
(21) Numéro de dépôt: 11799103.4
(22) Date de dépôt: 20.12.2011
(51) Int. Cl.: A61K 31/50, C07D 237/14

(54) **DERIVES DE DIARYLPYRIDAZINONES, LEUR PREPARATION ET LEUR APPLICATION EN THERAPEUTIQUE HUMAINE**
DIARYLPYRIDAZINONDERIVATE, IHRE HERSTELLUNG UND VERWENDUNG ZUR BEHANDLUNG VON MENSCHEN
DIARYLPYRIDAZINONE DERIVATIVES, PREPARATION THEREOF, AND USE THEREOF FOR THE TREATMENT OF HUMANS

(30) Priorité: 22.12.2010 FR 1061021
(43) Date de publication de la demande: 30.10.2013
(73) Titulaire: Pierre Fabre Médicament, 92100 Boulogne-Billancourt (FR)
(72) Inventeur: DUPONT-PASSELAIGUE, Elisabeth, 31770 Colomiers (FR); LE ROY, Isabelle, 31270 Frouzins (FR); MIALHE, Samuel, F-81100 Castres (FR); PIGNIER, Christophe, F-81100 Castres (FR)
(74) Mandataire: Regimbeau
(86) Numéro de dépôt international: PCT/EP2011/073476
(87) Numéro de publication internationale: WO 2012/085001

(56) Documents cités:
- WO-A1-98/41511
- WO-A1-2006/136304

## Description

La présente invention concerne des dérivés de diarylpyridazinones, leur préparation et leurs applications en thérapeutique humaine, en tant que bloqueurs des canaux potassiques Kv et plus particulièrement les canaux Kv1.5, Kv4.3 et Kv11.1.

Les canaux potassiques représentent la plus grande famille des canaux ioniques dans le génome humain avec environ 80 gènes (Tamargo et al, Cardiovasc. Res. 2004, 62 : 9-33). Ces canaux potassiques peuvent être subdivisés en 3 sous-familles : les canaux activés par le potentiel ou voltage (canaux Kᵥ) et le calcium (canaux K_{Ca}), les canaux à rectification entrante (Kᵢᵣ) et les canaux potassiques à 2 pores (K₂ₚ). La sous-famille des canaux activés par le potentiel est la plus représentée dans l'organisme humain avec une répartition quasi ubiquitaire dans les cellules excitables (cellules cardiaques, neurones, cellules musculaires striées ou lisses) et les cellules non-excitables comme les cellules pancréatiques, prostatiques, parathyroide etc. (pour revue, Gutman G et al, Pharmacol. Rev. 2005, 57: 473-508).

La fonction principale des canaux potassiques Kv dans les cellules excitables et le contrôle du potentiel de membrane de repos et de la durée du potentiel d'action (Nerbonne et Kass, Physiol. Rev.2005 ;85:1205-1253). A ce titre plusieurs canaux Kv interviennent dans ce contrôle tant au niveau des oreillettes que des ventricules cardiaques. Les canaux Kv4.3 associés aux sous unités KCHIP 2 forment le courant Iₜₒ qui intervient dans la phase de repolarisation précoce du potentiel d'action (PA), les canaux KVLQT1/MinK et hERG qui interviennent dans la phase tardive de repolarisation du PA (en générant respectivement les courants I_{Ks} et I_{Kr}). Les canaux pré-cités sont répartis uniformément entre les oreillettes et les ventricules cardiaques. Cependant, deux autres types de canaux potassiques montrent une répartition uniquement dans les oreillettes. Les canaux potassiques dépendant du potentiel (K_{v1.5}) responsables du courant I_{Kur} et les canaux à rectification entrante activés par l'acétylcholine (Kir3.1 et Kir3.4 responsable du courant I_{K-Ach}).

Des modifications de l'activité électrique membranaire sont observées dans de nombreuses pathologies, notamment les pathologies cardiaques de troubles du rythme. Parmi celles-ci, la fibrillation atriale (FA) est un trouble du rythme grave qui correspond à une activité totalement désynchronisée des mycocytes atriaux résultant en une activité électrique ininterrompue, rapide et irrégulière. La FA est induite par l'apparition de circuits électriques de réentrée dans le tissu atrial (Miyasaka Y et al, Circulation 2006, 114 : 119-125). Il n'existe pas à ce jour de traitement anti-arythmique spécifique de l'étage atrial pour réduire l'incidence de la FA ce qui constitue donc un besoin médical important (Page et Roden, Nat. Rev. Drug Discov. 2005, 4 : 899-910).

La présence d'une multitude de circuits de micro-réentrée activés simultanément explique le caractère anarchique de l'activité électrique constatée aussi bien par voie endocavitaire que sur l'ECG. Ce trouble rythmique se développe généralement sur un myocarde atrial pathologique au plan électrophysiologique, dont les périodes réfractaires sont trop courtes et très inégales entre elles, donc très vulnérables à la moindre extrasystole. Ces anomalies s'inscrivent dans le cadre d'un phénomène de remodelage myocardique, consécutif à une surcharge de pression ou à un étirement provoquant des altérations morphologiques (hypertrophie, dilatation, fibrose) ainsi que des modifications dans la régulation des courants ioniques transmembranaires, modifiant les caractéristiques électrophysiologiques des myocytes atriaux. Étant donné que chaque accès de FA entretient, voire aggrave ce processus de remodelage mécanique et électrophysiologique, on comprend alors que la FA possède un fort potentiel de récidive et que son évolution naturelle se fasse vers la chronicité. À l'opposé, il a été identifié récemment des FA de type focal, prenant leur origine en un point précis qui se trouve presque toujours être une extension du myocarde atrial dans les veines pulmonaires. Ces cas assez rares de FA prennent un caractère assez monomorphe, en tout cas comparable aux extrasystoles atriales initiant l'accès ou constatées de façon intermittente entre les crises. Dans tous les cas, la perte de la systole atriale a pour conséquence une chute du débit cardiaque variant entre 20 et 30 % et d'autant plus importante que celui-ci est diminué à l'état basal. Parallèlement, l'existence d'une stase sanguine dans les cavités atriales, notamment dans certains cul-de-sac tels que les auricules, rend compte du risque thromboembolique. Toutefois, le risque embolique n'est que partiellement conditionné par la seule présence de la FA, la stase atriale étant aussi liée à l'augmentation des pressions intra cavitaires (dysfonction ventriculaire gauche systolique ou diastolique, valvulopathie ou prothèse valvulaire).

Le remodelage électrique constitue donc le substrat majeur de la genèse de la FA, il résulte d'une diminution de l'activité des canaux calciques de type L laissant les canaux potassiques Kv1.5 exercer pleinement leur rôle repolarisant au travers du courant potassique ultra rapide (Bhakta et Miller, Expert Opin. Ther. Targets 2007, 11 : 1161-1178). La conséquence est un raccourcissement dramatique de la période réfractaire ce qui constitue le facteur déclenchant des micro-réentrées. Sachant que les canaux potassiques Kv1.5 ne sont pas exprimés de façon fonctionnelle à l'étage ventriculaire, un bloqueur de ces canaux constituera donc un anti-arythmique sélectif de l'étage atrial sans affecter l'électrophysiologie ventriculaire. Son effet pharmacologique se traduit par un allongement de la période réfractaire et donc une moindre incidence des circuits de micro-réentrée. Un certain nombre de données expérimentales obtenues avec des produits de référence confirment l'intérêt du blocage de Kv1.5 comme cible thérapeutique (Gögelein et al, Naunyn Schmiedeberg's Arch Pharmacol 2004, 370 : 183-192, Regan et al, J Pharmacol Exp Ther 2008, 324 : 322-330).

Les changements rapides du potentiel de membrane sont bien connus dans les cellules excitables mais des variations lentes du potentiel sont observées dans toutes les cellules et sont associées au contrôle du cycle cellulaire. Le cycle cellulaire est un paramètre clé dans le comportement cellulaire qui doit être régulé et coordonné pour le développement, la régénération tissulaire et la prolifération cellulaire (Pardo, Physiology, 2004 ;19 :285-292 ; Blackistion et al, Cell Cylce, 2009 ;8-21 : 3527-3536). D'une manière générale, le blocage des canaux potassiques conduit à une diminution de la prolifération dans des modèles physiologiques (comme dans les lymphocytes) et pathologiques (cancer). Le rôle des canaux potassiques dans la régulation du cycle cellulaire a été démontré dans de nombreux types cellulaires, qu'ils soient physiologiques ou pathologiques (lignées ou tumeurs cancéreuses) provenant de mélanome humain, le cancer des poumons, lymphome, mésotheliome, hépatocarcinome, lymphocytes, monocytes (pour revue Pardo et al, J. Membr. Biol, 2005 ;205 : 115-124).

Comme utilisé précédemment, le terme « Kv » indique la famille des canaux potassiques dépendant du potentiel et comprend différentes sous familles (Kv1., Kv2., Kv3....) parmi lesquels on retrouve les canaux Kv1.1, Kv1.2, Kv1.3...

« Un bloqueur des canaux Kv » dénote une molécule qui réduit ou bloque le flux des ions K⁺ au travers du canal.

On notera le document WO 2006/136304, qui divulgue des pyridazinones ayant une activité de bloqueur des canaux potassiques Kv, et plus particulièrement des canaux potassiques de type Kv1.5, mais ces composés diffèrent par au moins deux caractéristiques structurelles par rapport à ceux de la présente invention.

Par ailleurs, le document WO 98/41511 divulgue des pyridazinones présentant une activité inhibitrice de la cyclooxygénase-2, utile pour le traitement des maladies inflammatoires. En particulier, le composé 25 présente un groupe phényle substitué par un groupe SO₂Me.Comme utilisé ici, le terme « sels » désigne les sels inorganiques d'addition d'acides et de bases des composés de la présente invention. De préférence, les sels sont pharmaceutiquement acceptables, c'est à dire qu'ils sont non-toxiques pour le patient auquel ils sont administrés. Le terme « pharmaceutiquement acceptable » se réfère à des entités moléculaires et des compositions qui ne produisent aucun effet adverse, allergique ou autre réaction indésirable quand elles sont administrées à un animal ou un humain. Quand utilisé ici, le terme « excipient pharmaceutiquement acceptable » inclut tout diluant, adjuvant ou excipient, tels que des agents préservatifs, des agents de remplissage, des agents désintégrant, mouillant, émulsifiant, dispersant, antibactérien ou antifongique, ou bien encore des agents qui permettraient de retarder l'absorption et la résorption intestinale et digestive. L'utilisation de ces milieux ou vecteurs est bien connue de l'art. Excepté si l'agent est chimiquement incompatible avec un dérivé diarylpyridazinone, son utilisation dans des compositions pharmaceutiques avec les composés selon l'invention est envisagée. Dans le contexte de l'invention, le terme « traitement », comme utilisé ici, signifie empêcher ou inhiber la survenue ou la progression de l'affection à laquelle le terme s'applique, ou bien d'un ou de plusieurs symptômes de cette affection.

La présente invention a pour objet des dérivés de diarylpyridazinones bloqueurs des canaux potassiques Kv (plus particulièrement les canaux Kv1.5, Kv4.3 et Kv11.1) et leur application en thérapeutique humaine.

Ces composés correspondent à la formule générale I : 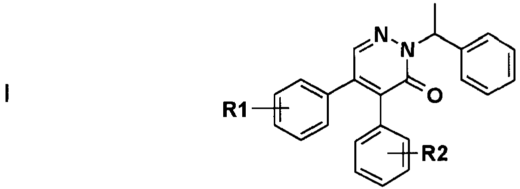 dans laquelle
R₁ et R₂ représentent de manière simultanée ou indépendamment un ou plusieurs groupements choisis parmi : halogène tel que F, Br, Cl, alkyle linéaire ou branché en C₁-C₄, hydroxy, alkoxy linéaire ou branché en C₁-C₄, nitrile ou arylsulfonamido dont l'aryl est éventuellement substitué par un groupement alkyle linéaire ou branché en C₁-C₄,
ainsi que les différents énantiomères et leurs mélanges en toutes proportions, et leurs sels pharmaceutiquement acceptables.

Dans le cadre de la présente invention, le groupe aryle désigne des monocycles aromatiques hydrocarbonés de 5 ou 6 chaînons.

Selon un mode de réalisation de l'invention, les composés de formule générale I sont ceux pour lesquels : 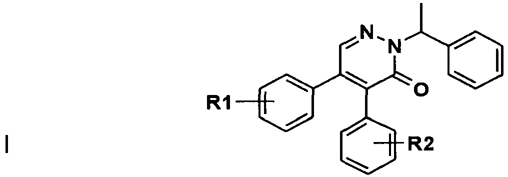
R₁ représente un groupement hydroxy, méthoxy ou cyano ;
R₂ représente plusieurs groupements choisis parmi : halogène tel que F, Br, Cl, alkyle linéaire ou branché en C₁-C₄, hydroxy, alkoxy linéaire ou branché en C₁-C₄, nitrile ;
ainsi que les différents énantiomères et leurs mélanges en toutes proportions, et leurs sels pharmaceutiquement acceptables.

Selon un autre mode de réalisation de l'invention, les composés de formule générale I sont ceux pour lesquels : 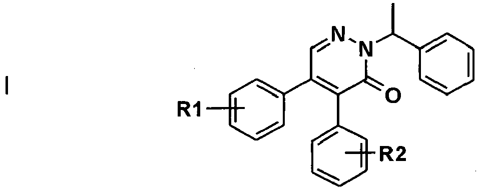
R₁ représente un groupement hydroxy,
R₂ représente plusieurs groupements choisis parmi : halogène tel que F, Cl, alkyle linéaire ou branché en C₁-C₄, hydroxy, alkoxy linéaire ou branché en C₁-C₄, nitrile ;
ainsi que les différents énantiomères et leurs mélanges en toutes proportions, et leurs sels pharmaceutiquement acceptables.

Selon un autre mode de réalisation de l'invention, les composés de formule générale I sont ceux pour lesquels : 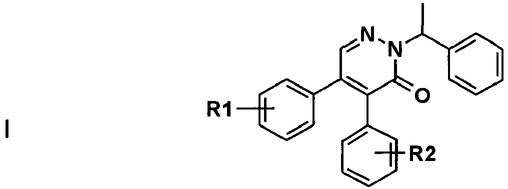
R₁ représente un groupement hydroxy situé en position para (position 4) sur le phényl qu'il substitue,
R₂ représente plusieurs groupements choisis parmi : Cl, méthyle, hydroxy, méthoxy, nitrile,
ainsi que les différents énantiomères et leurs mélanges en toutes proportions, et leurs sels pharmaceutiquement acceptables.

La présente invention concerne les composés de formule générale I caractérisés en ce qu'ils sont choisis parmi :
1. 4,5-Bis-(4-hydroxy-phényl)-2-(1-phényl-éthyl)-2H-pyridazin-3-one
2. 4,5-Bis-(4-hydroxy-phényl)-2-((S)-1-phényl-éthyl)-2H-pyridazin-3-one
3. 4,5-Bis-(4-hydroxy-phényl)-2-((R)-1-phényl-éthyl)-2H-pyridazin-3-one
4. 2,2'-(6-oxo-1-(1-phényléthyl)-1,6-dihydropyridazine-4,5-diyl)dibenzonitrile
5. 3,3'-(6-oxo-1-(1-phényléthyl)-1,6-dihydropyridazine-4,5-diyl)dibenzonitrile
6. 4,5-Bis-(4-méthoxy-phényl)-2-(1-phényl-éthyl)-2H-pyridazin-3-one
7. N,N'-(3,3'-(6-oxo-1-(1-phényléthyl)-1,6-dihydropyridazine-4,5-diyl)bis(3,1-pheylee))bis(4-méthylbenzenesulfonam ide)
8. 3-(5-(4-méthoxyphényl)-6-oxo-1-(1-phényléthyl)-1,6-dihydropyridazin-4-yl)benzonitrile
9. 2-[5-(4-Méthoxy-phényl)-6-oxo-1-(1-phényl-éthyl)-1,6-dihydro-pyridazin-4-yl]-benzonitrile
10. N-{3-[5-(3,4-Diméthyl-phényl)-6-oxo-1-(1-phényl-éthyl)-1,6-dihydro-pyridazin-4-yl]-phényl}-4-méthyl-benzenesulfonamide
11. 4,5-Bis-(3,4-dichloro-phényl)-2-(1-phényl-éthyl)-2H-pyridazin-3-one

La présente invention s'étend aussi aux différents énantiomères des composés de formule générale I, ainsi que leurs mélanges en toutes proportions.

Les mélanges des énantiomères en toutes proportions incluent également les mélanges racémiques.

L'objet de l'invention concerne également les différents énantiomères et leurs mélanges en toutes proportions des composés de formule générale I ainsi que les sels pharmaceutiquement acceptables.

La présente invention s'étend également aux procédés de préparation chimique des composés de formule générale I ainsi que les différents énantiomères et leurs mélanges en toutes proportions.

La préparation des deux énantiomères peut être réalisée de manière énantiosélective à partir respectivement des (R) ou (S)-1-phényléthanols. Par ailleurs, à partir du racémique il est possible d'obtenir les deux énantiomères par séparation par HPLC préparative sur colonne chirale (par exemple Chiralpack AD-H, éluent : heptane/EtOH/diéthylamine).

La présente invention concerne également les composés de formule générale I ainsi que les différents énantiomères et leurs mélanges en toutes proportions, et leurs sels pharmaceutiquement acceptables pour leur utilisation en tant que bloqueurs de canaux potassiques Kv et plus particulièrement les canaux Kv1.5, Kv4.3 et Kv11.1.

La présente invention concerne également les composés de formule générale I ainsi que les différents énantiomères et leurs mélanges en toutes proportions, et leurs sels pharmaceutiquement acceptables pour leur utilisation en tant que médicament.

L'invention concerne aussi les composés de formule générale I ainsi que les différents énantiomères et leurs mélanges en toutes proportions, et leurs sels pharmaceutiquement acceptables pour leur utilisation en tant que médicament destiné au traitement et/ou la prévention des maladies nécessitant des bloqueurs de canaux potassiques Kv et plus particulièrement les canaux Kv1.5, Kv4.3 et Kv11.1.

L'invention concerne aussi les composés de formule générale I ainsi que les différents énantiomères et leurs mélanges en toutes proportions, et leurs sels pharmaceutiquement acceptables pour leur utilisation en tant que médicament destiné au traitement et/ou la prévention des maladies telles que la fibrillation atriale et les troubles du rythme cardiaques des oreillettes et/ou des ventricules mais également des pathologies dans lesquelles le cycle cellulaire, la prolifération cellulaire et la regénération sont modifiés (cancer, inflammation chronique).

L'invention s'étend également aux compositions caractérisées en ce qu'elles contiennent à titre de principe actif un composé de formule générale I ou l'un de ses énantiomères et leurs mélanges en toutes proportions, ou l'un de ses sels pharmaceutiquement acceptable.

L'invention concerne également une composition pharmaceutique caractérisée en ce qu'elle contient un composé de formule générale I ou l'un de ses énantiomères et leurs mélanges en toutes proportions, ou l'un de ses sels pharmaceutiquement acceptable en association avec tout excipient pharmaceutiquement acceptable.

Les compositions pharmaceutiques selon l'invention peuvent être administrées par voie orale, sublinguale, sous-cutanée, intramusculaire, intraveineuse, transdermique, locale ou rectale. Dans ce cas l'ingrédient actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, topique, intramusculaire, intraveineuse, intra-nasale ou intraoculaires et les formes d'administration rectale. Les formulations appropriées pour la forme d'administration choisie sont connues par l'homme du métier et décrites, par exemple dans : Remington, The science and Practice of Pharmacy, 19ème édition, 1995, Mack Publishing Company.

Les dosages des composés de formule I dans les compositions de l'invention peuvent être ajustés afin d'obtenir une quantité de substance active qui est efficace pour obtenir la réponse thérapeutique désirée pour une composition particulière à la méthode d'administration. La dose efficace d'un composé de l'invention varie en fonction de nombreux paramètres tels que, par exemple, la voie d'administration choisie, le poids, l'âge, le sexe, la nature de la pathologie et la sensibilité de l'individu à traiter. En conséquence, la posologie optimale devra être déterminée en fonction des paramètres jugés pertinents, par le spécialiste en la matière.

### SYNTHESE

Les composés de la présente invention peuvent être synthétisés en utilisant les voies de synthèse décrites ci-dessous ou en utilisant des méthodes de synthèse connues de l'homme de métier.

Cette méthode de synthèse des composés de formule générale I (schéma 1) est caractérisée en ce que l'on condense une dibromo ou dichloro pyridazinone de formule générale II pour lequel X représente soit un atome chlore ou un atome de brome, 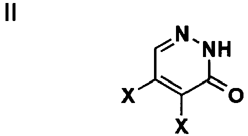 avec un dérivé de formule générale III, 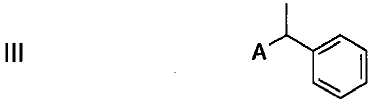 pour lequel
- lorsque A représente un atome d'halogène tel qu'un atome chlore ou un atome de brome, on utilise une base telle que Cs₂CO₃ dans un solvant tel que le diméthylformamide.
- lorsque A représente OH, on utilise des conditions de couplage de Mitsunobu telles qu'en présence de diéthylazodicarboxylate d'éthyle et de triphénylphosphine dans un solvant tel que le THF. Ces conditions sont en particulier applicables à la synthèse énantiosélective des composés de formule générale I à partir du (R) ou (S)-1-phényléthanol.

L'intermédiaire IV obtenu 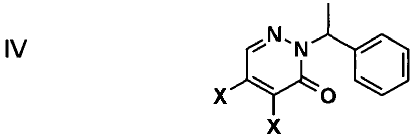 est alors couplé (étape 1) avec un dérivé du bore V 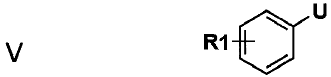 pour lequel R1 est tel que défini dans la formule générale I et U représente B(OH)₂ ou 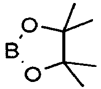 dans un mélange de solvants tel que toluène/éthanol ou eau/acétonitrile ou dioxane/eau en présence d'une base telle que le carbonate de sodium ou de potassium et d'un catalyseur tel que le tétrakis(triphénylphosphine)palladium ou PdCl₂/2PPh₃.

Ces conditions opératoires conduisent majoritairement à la formation du composé VI et minoritairement à la formation du composé VII.

L'intermédiaire VI est alors remis en réaction (étape 2) :
- soit avec le dérivé du bore V dans les conditions de couplage décrites précédemment pour conduire à la formation du composé VII.
- soit avec le dérivé du bore VIII 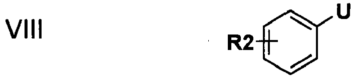 pour lequel R₂ est tel que défini dans la formule générale I et U est tel que défini précédemment dans les conditions de couplage décrites précédemment pour l'étape 1 pour conduire à la formation du composé IX.

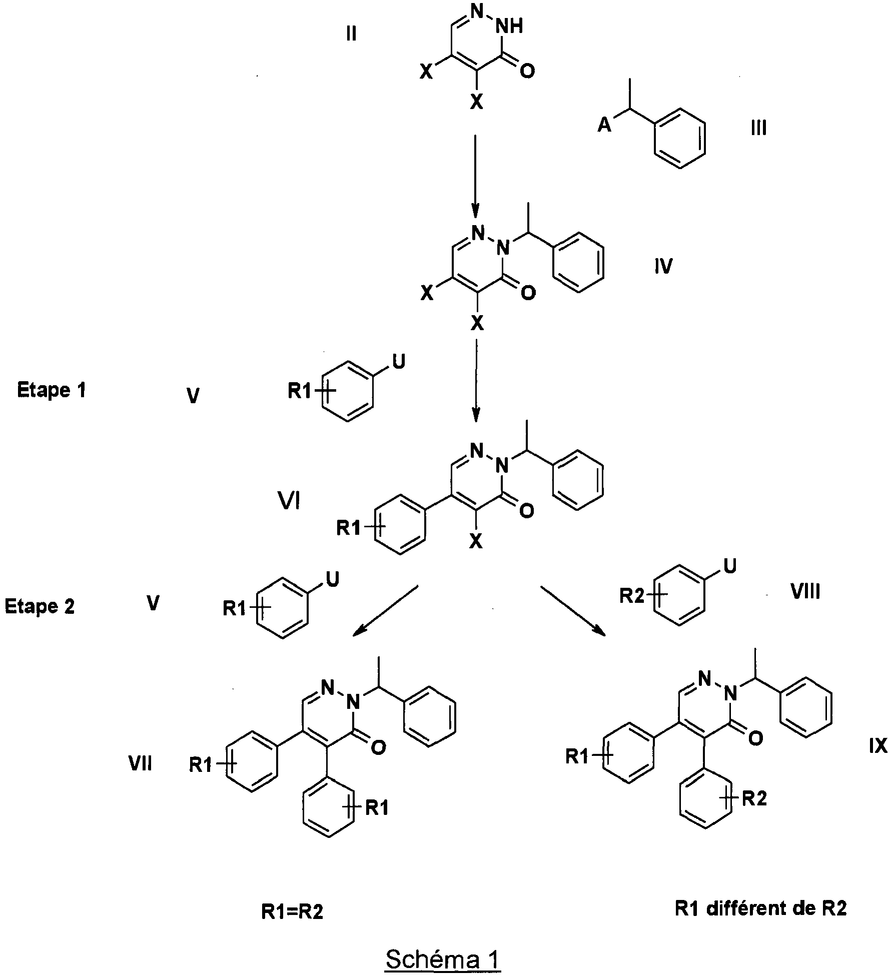

Les composés intermédiaires et finaux peuvent être, si on le désire, purifiés suivant une ou plusieurs méthodes de purification choisies parmi, l'extraction, la filtration, la chromatographie sur gel de silice, l'HPLC préparative sur phase normale ou inverse ou chirale, la cristallisation.

Les matières premières utilisées dans les procédés décrits précédemment sont commerciales ou aisément accessibles à l'homme de métier selon des procédés décrits dans la littérature.

Les exemples suivants illustrent l'invention sans en limiter la portée.

Les analyses élémentaires et les spectres de masse et RMN confirment les structures des composés.

### EXEMPLES

### A) INTERMEDIAIRES

### Intermédiaires 1:

### a) 4,5-dichloro-2-(1-phényléthyl)pyridazin-3(2H)-one(1a)

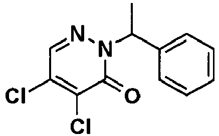

La 4,5-dichloropyridazin-3(2H)-one (20g, 121mmol) est placée en présence de 1-bromoéthyl)benzene (33,7g, 182mmol) et de carbonate de césium (47,4g, 145mmol) dans 100mL de DMF à température ambiante pendant 4h. Après concentration à sec, le résidu est repris par l'eau et extrait à l'acétate d'éthyle. Les phases organiques sont séchées puis concentrées à sec. Le résidu obtenu est purifié par chromatographie flash sur silice (Ether de pétrole-AcOEt: 95-5). 31g d'huile claire sont obtenus (rendement 95%). *CCM gel de silice 60 F 254Merck,* Ether de pétrole-AcOEt: 90-10, *Rf=0,50.*

### b) 4,5-Dichloro-2-((S)-1-phényl-éthyl)-2H-pyridazin-3-one (1b)

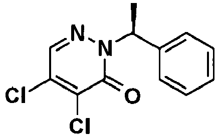

La 4,5-dichloropyridazin-3(2H)-one (1,35g, 8,2mmol) est placée dans 30mL de THF en présence de (R)-1-phényléthanol (1g, 8,2mmol) et de triphénylphosphine (2,15g, 8,2mmol) auxquels est ajouté du diéthylazodicarboxylate d'éthyle (1,71g, 9,82mmol. Le milieu réactionnel est agité une nuit à température ambiante puis concentré à sec. Le résidu est repris par l'eau et extrait au dichlorométhane sur colonne SPE (terres de diatomées). Les phases organiques sont concentrées à sec et le résidu obtenu est par chromatographie flash sur silice (CH₂Cl₂). 2,1 g d'huile jaune sont isolés (rendement 80%). *CCM gel de silice 60 F 254 Merck, CH₂Cl₂-MeOH:95-5, Rf=0, 66.*

### c) 4,5-Dichloro-2-((R)-1-phényl-éthyl)-2H-pyridazin-3-one (1c)

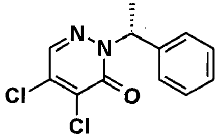

L'intermédiaire 1c (huile) est préparé à partir de (S)-1-phényléthanol selon le mode opératoire décrit pour l'intermédiaire 1 b (77%). *CCM gel de silice 60 F 254 Merck, CH₂Cl₂-MeOH:90-10, Rf=0,82.*

### B) COMPOSES SELON L'INVENTION

### Exemple 1 : 4,5-Bis-(4-hydroxy-phényl)-2-(1-phényl-éthyl)-2H-pyridazin-3-one (1)

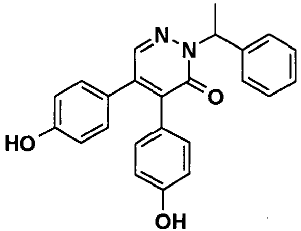

Le composé 1 est préparé selon la méthode de synthèse suivante :
Etape 1 : l'intermédiaire 1a (8,7g, 32,3mmol) est placé en présence de tétrakis(triphénylphosphine)palladium(0) (1,12g, 0,97mmol) et de carbonate de sodium (6,85g, 64,7mmol) dans un mélange de 50mL de toluène et 50 mL d'éthanol et le mélange porté à 80°C. 1,2 équivalent d'acide 4-hydroxyphénylboronique sont additionnés et le mélange est porté à reflux pendant 5h puis 1,2 équivalent supplémentaire d'acide 4-hydroxyphénylboronique est additionné et le reflux est maintenu toute la nuit. Après concentration à sec, le résidu est repris à l'eau et extrait à l'AcOEt. Après séchage des phases organiques et concentration à sec, le résidu obtenu est purifié par chromatographie flash (CH₂Cl₂-MeOH, gradient 100-0 à 97-3 sur 40 min). 0,7g de composé 1 minoritaire est obtenu et 8,2g de solide correspondant au produit mono substitué 4-chloro-5-(4-hydroxyphényl)-2-(1-phényléthyl)-pyridazin-3(2H)-one majoritaire est obtenu (rendement:78%).
Etape 2: Ce produit mono substitué est remis en réaction dans les conditions décrites pour l'étape 1 (2,4 équivalents d'acide 4-hydroxyphénylboronique, reflux 1 nuit). Après traitement du milieu réactionnel, le résidu obtenu est purifié par chromatographie flash (CH₂Cl₂-MeOH, gradient 100-0 à 98-2 sur 20 min). Le résidu est trituré dans un mélange diéthyléther-CH₂Cl₂-MeOH : 40-5-2 et le composé 1 (solide) obtenu est isolé par filtration (7,2g, rendement 78%).
   CCM gel de silice 60 F 254 Merck, CH₂Cl₂-MeOH : 95-5, Rf=0,35.
   F=160°C
   RMN ¹H (DMSO-d6) ppm: 9,56 (m, 2H), 8,02 (s, 1 H), 7,39 (m, 5H), 6,96 (m, 4H), 6,63 (m, 4H), 6,24 (m,1H), 1,72 (d, 3H).
   MS (+ESI) m/z 385 (MH+)

### Exemple 2 : 4,5-Bis-(4-hydroxy-phényl)-2-((S)-1-phényl-éthyl)-2H-pyridazin-3-one (2)

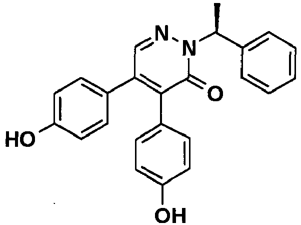

Le composé 2 est préparé selon la méthode de synthèse décrite pour l'exemple 1 à partir de l'intermédiaire 1c (rendement :85%).
CCM gel de silice 60 F 254 Merck, CH₂Cl₂-MeOH : 90-10, Rf=0,60.
F=168°C
RMN ¹H (DMSO-d6) ppm: 9,70 (s, 1 H), 9,54 (s, 1 H), 8,02 (s, 1 H), 7,39 (m, 5H), 6,97 (m, 4H), 6,63 (m, 4H), 6,24 (m,1H), 1,72 (d, 3H).
MS (+ESI) m/z 385 (MH+)
α_{calc} (MeOH)= -256.5°
HPLC chirale : colonne Chiralpack AD-H 250*4,6mm DAI, éluant (1mL/min): heptane /EtOH/diéthylamine : 80/20/0,1 , temps de rétention :8,92min.

### Exemple 3 : 4,5-Bis-(4-hydroxy-phényl)-2-((R)-1-phényl-éthyl)-2H-pyridazin-3-one (3)

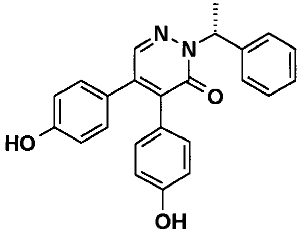

Le composé 3 est préparé selon la méthode de synthèse décrite pour l'exemple 1 à partir de l'intermédiaire 1 b (rendement :43%).
CCM gel de silice 60 F 254 Merck, CH₂Cl₂-MeOH : 90-10, Rf=0,60.
F=222°C
RMN ¹H (DMSO-d6) ppm: 9,70 (s, 1 H), 9,54 (s, 1 H), 8,02 (s, 1 H), 7,39 (m, 5H), 6,97 (m, 4H), 6,63 (m, 4H), 6,24 (m,1H), 1,72 (d, 3H).
MS (+ESI) m/z 385 (MH+)
α_{calc} (MeOH)= 272,2°
HPLC chirale : colonne Chiralpack AD-H 250*4,6mm DAI, éluant (1mL/min): heptane /EtOH/ diéthylamine : 80/20/0,1 , temps de rétention :7,23min.

### Exemple 4 : 2,2'-(6-oxo-1-(1-phényléthyl)-1,6-dihydropyridazine-4,5-diyl)dibenzonitrile (4)

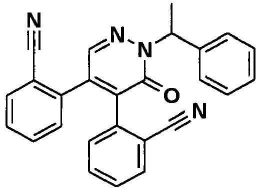

Le composé 4 est préparé à partir de l'intermédiaire 1a et du 2-(4,4,5,5-tetraméthyl-1,3,2-dioxaborolan-2-yl)benzonitrile selon l'étape 1 de la méthode de synthèse en utilisant PdCl₂/2PPh₃, Na₂CO₃ et un mélange eau/acétonitrile : 1/1. Le produit minoritaire formé correspond au composé 4 (rendement :3,4%).
CCM gel de silice 60 F 254 Merck, CH₂Cl₂, Rf=0,23.
F=200°C
RMN ¹H (DMSO-d6) ppm: 8,19 (s, 1 H), 7,80 (d, 2H), 7,75 (d, 2H), 7,36 (m, 9H), 6,27 (q, 1 H), 1,76 (d, 3H).
MS (+ESI) m/z 403 (MH+)

### Exemple 5 : 3,3'-(6-oxo-1-(1-phényléthyl)-1,6-dihydropyridazine-4,5-diyl)dibenzonitrile (5)

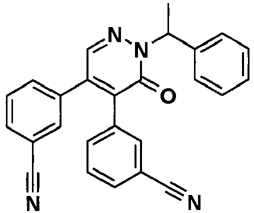

Le composé 5 est préparé à partir de l'intermédiaire 1a et de l'acide 3-cyanophénylboronique dans les conditions décrites pour l'exemple 4. Le produit minoritaire formé (solide) correspond au composé 4 (rendement :7,4%).
CCM gel de silice 60 F 254 Merck, CH₂Cl₂, Rf=0,11.
F=202°C
RMN ¹H (DMSO-d6) ppm: 8,23 (s, 1 H), 7,78 (m, 3H), 7,72 (s, 1 H), 7,40 (m, 9H), 6,28 (q, 1 H), 1,77 (d, 3H).
MS (+ESI) m/z 403 (MH+)

### Exemple 6 : 4,5-Bis-(4-méthoxy-phényl)-2-(1-phényl-éthyl)-2H-pyridazin-3-one (6)

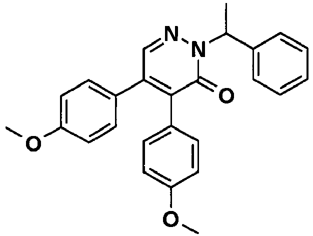

Le composé 6 est préparé à partir de l'intermédiaire 1 a et de l'acide 4-méthoxyphénylboronique dans les conditions décrites pour l'exemple 1 en utilisant le tétrakis(triphénylphosphine)palladium(0), K₂CO₃ et un mélange dioxane/eau: 3/1. Le composé 6 est isolé sous forme de solide (rendement :71%).
CCM gel de silice 60 F 254 Merck, Ether de pétrole-AcOEt:80-20, Rf=0,20. RMN ¹H (CDCl₃) ppm: 7,88 (s, 1H), 7,53 (d, 2H), 7,37-7,31 (m, 2H), 7,30-7,26 (m, 1H), 7,14-7,13 (d, 2H), 7,06-7,02 (d, 2H), 6,80-6,75 (m, 4H), 6,47-6,40 (m, 1H), 3,78 (s, 3H), 3,77 (s, 3H), 1,83 (s, 3H).
MS (+ESI),m/z 413 (MH+)

### Exemple 7 : N,N'-(3,3'-(6-oxo-1-(1-phényléthyl)-1,6-dihydropyridazine-4,5-diyl)bis(3,1-phénylene))bis(4-méthylbenzenesulfonamide) (7)

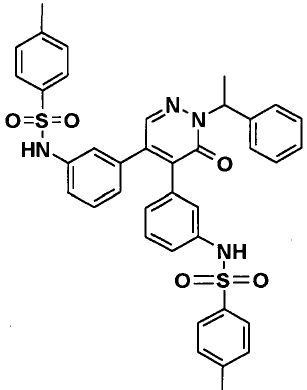

Le composé 7 est préparé à partir de l'intermédiaire 1 a et du 4-méthyl-N-(3-(4,4,5,5-tetraméthyl-1,3,2-dioxaborolan-2-yl)phényl)benzenesulfonamide dans les conditions décrites pour l'exemple 6. Le composé 7 est isolé sous forme de solide (rendement :74%).
CCM gel de silice 60 F 254 Merck, Ether de pétrole-AcOEt:50-50, Rf=0,46.
F=202°C
RMN ¹H (DMSO) ppm: 10,26 (s, 1 H), 10,14 (s, 1 H), 7,92 (s, 1 H), 7,53 (d, 4H), 7,42-7,25 (m, 9H), 7,08-6,88 (m, 6H), 6,52 (dd, 2H), 6,26-6,18 (m, 1H), 2,31 (s, 6H), 1,74 (d, 3H).
MS (+ESI) m/z 691 (MH+)

### Exemple 8 : 3-(5-(4-méthoxyphényl)-6-oxo-1-(1-phényléthyl)-1,6-dihydropyridazin-4-yl)benzonitrile (8)

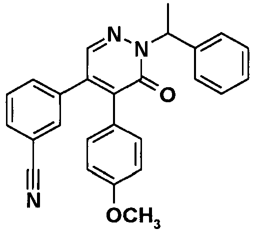

Le composé 8 est préparé à partir de l'intermédiaire 1a et de l'acide 3-cyanophénylboronique selon l'étape 1 de la méthode de synthèse en utilisant PdCl₂/2PPh₃, Na₂CO₃ et un mélange eau/acétonitrile : 1/1. Le produit majoritaire formé (1,96g, 3-(5-chloro-6-oxo-1-(1-phényléthyl)-1,6-dihydropyridazin-4-yl)benzonitrile, rendement: 19%) est isolé puis engagé dans l'étape 2 de la méthode de synthèse en utilisant l'acide 4-méthoxyphénylboronique avec le tétrakis(triphénylphosphine)palladium(0), K₂CO₃ et un mélange dioxane/eau: 2/1. Le composé 8 est isolé sous forme de solide (rendement :62%).
CCM gel de silice 60 F 254 Merck, Ether de pétrole-AcOEt:50-50, Rf=0,53.
F=198°C
CCM gel de silice 60 F 254 Merck, Ether de pétrole-AcOEt: 50-50, Rf=0,53. RMN ¹H (DMSO-d6) ppm: 8,13 (s, 1H), 7,77 (m, 2H), 7,38 (m, 7H), 7,09 (d, 2H), 6,81 (d, 2H), 6,28 (q, 1 H), 3,71 (s, 3H), 1,75 (d, 3H).
MS (+ESI) m/z 408 (MH+)

### Exemple 9 : 2-[5-(4-Méthoxy-phényl)-6-oxo-1-(1-phényl-éthyl)-1 ,6-dihydro-pyridazin-4-yl]-benzonitrile (9)

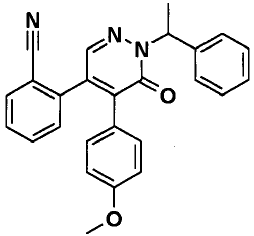

Le composé 9 est préparé à partir de l'intermédiaire 1a et du 2-(4,4,5,5-tetraméthyl-1,3,2-dioxaborolan-2-yl)benzonitrile selon l'étape 1 de la méthode de synthèse en utilisant PdCl₂/2PPh₃, Na₂CO₃ et un mélange eau/acétonitrile : 1/1. Le produit majoritaire formé (1,5g, 2-(5-chloro-6-oxo-1-(1-phényléthyl)-1,6-dihydropyridazin-4-yl)benzonitrile, rendement : 16%) est isolé puis engagé dans l'étape 2 de la méthode de synthèse avec l'acide 4-méthoxyphénylboronique en utilisant le tétrakis(triphénylphosphine)palladium(0), K₂CO₃ et un mélange dioxane/eau: 2/1. Le composé 9 est isolé sous forme de solide (rendement :71%).
CCM gel de silice 60 F 254 Merck, Ether de pétrole-AcOEt-70-30, Rf=0,45.
F=176°C
CCM gel de silice 60 F 254 Merck, Ether de pétrole-AcOEt: 50-50, Rf=0,53. RMN ¹H (DMSO-d6) ppm: 8,09 (s, 1H), 7,79 (d, 2H), 7,40 (m, 7H), 7,08 (d, 2H), 6,81 (d, 2H), 6,28 (q, 1H), 3,71 (s, 3H), 1,75 (d, 3H).
MS (+ESI) m/z 408 (MH+)

### Exemple 10 : N-{3-[5-(3,4-Diméthyl-phényl)-6-oxo-1-(1-phényl-éthyl)-1,6-dihydro-pyridazin-4-yl]-phényl}-4-méthyl-benzenesulfonamide (10)

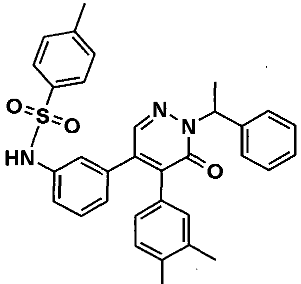

Le composé 10 est préparé à partir de l'intermédiaire 1a et du 4-méthyl-N-(4-(4,4,5,5-tetraméthyl-1,3,2-dioxaborolan-2-yl)phényl)benzenesulfonamide selon l'étape 1 de la méthode de synthèse en utilisant PdCl₂/2PPh₃, Na₂CO₃ et un mélange eau/acétonitrile : 1/1. Le produit majoritaire formé (-N-(3-(5-chloro-6-oxo-1-(5g, 1-phényléthyl)-1,6-dihydropyridazin-4-yl)phényl)-4-méthylbenzene-sulfonamide, rendement : 62%) est isolé puis engagé dans l'étape 2 de la méthode de synthèse avec l'acide 3,4-diméthylphénylboronique en utilisant le tétrakis(triphénylphosphine)palladium(0), K₂CO₃ et un mélange dioxane/eau: 2/1. Le composé 10 est isolé sous forme de solide (rendement :67%).
CCM gel de silice 60 F 254 Merck, CH₂Cl₂-MeOH:97,5-2,5, Rf=0,65.
RMN ¹H (DMSO) ppm: 10,24 (s, 1 H), 7,92 (s, 1 H), 7,54 (d, 2H), 7,41-7,26 (m, 7H), 7,10 (t, 1 H), 7,04 (s, 1 H), 6,96-6,91 (m, 2H), 6,88 (d, 1 H), 6,72 (d, 1 H), 6,62 (d, 1 H), 6,27-6,20 (m, 1H), 2,34 (s, 3H), 2,15 (s, 3H), 2,06 (s, 3H), 1,73 (d, 3H).
MS (+ESI) m/z 550 (MH+)

### Exemple 11 : 4,5-Bis-(3,4-dichloro-phényl)-2-(1-phényl-éthyl)-2H-pyridazin-3-one (11)

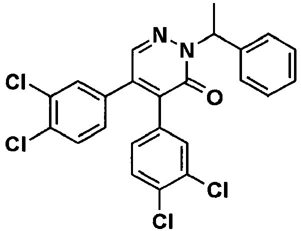

Le composé 11 est préparé à partir de l'intermédiaire 1 a et de l'acide 3,4-dichlorophénylboronique dans les conditions décrites pour l'exemple 1 en utilisant le tétrakis(triphénylphosphine)palladium(0), K₂CO₃ et un mélange dioxane/eau: 7/3. Le composé 12 est isolé sous forme de solide (rendement :54%).
F=92°C
CCM gel de silice 60 F 254 Merck, Ether de pétrole-AcOEt:80-20, Rf=0,54. RMN ¹H (DMSO) ppm: 8,19 (s, 1H), 7,64 (d, 1H), 7,61-7,57 (m, 2H), 7,54 (d, 1 H), 7,44-7,26 (m, 5H), 7,13 (dd, 1H), 7,07 (dd, 1H), 6,30-6,22 (m, 1H), 1,75 (d, 3H)
MS (+ESI) m/z 491 (MH+)

### C) EVALUATION PHARMACOLOGIQUE

L'évaluation pharmacologique des composés sur le canal potassique Kv1.5 a été réalisée en plaque 96 puits en technologie FLIPR par la mesure d'ion thallium.

Les cellules HEK293, transfectées de manière stable avec l'isoforme humaine des canaux Kv1.5, sont ensemencées 24h avant l'expérimentation dans des plaques 96 puits (15 10⁶ cellules /plaque, 200 µl / puit) polylysinées dans le milieu de culture suivant : DMEM, 10% SVF, Penicilline/Streptomycine, G418 en tant qu'antibiotique de sélection.

L'expérimentation en FLIPR est réalisée à l'aide du « FLIPR Potassium Ion Channel Assay Kit) comme indiquée par le manufacturier (Molecular Devices).

Brièvement, Le milieu de culture est remplacé par la solution contenant le marqueur de thallium pendant 90min à 37°C. A l'issue de cette étape, les composés à tester sont ajoutés à une concentration finale de 10 µM dans le puits pendant 15 min à 37 °C. La fluorescence de base est alors lue pendant 60 sec. L'addition d'un milieu dépolarisant (20 mM de potassium et 3 mM de thallium final), ouvre les canaux potassiques et induit une augmentation de la fluorescence du fluorophore thallium correspondant à un influx d'ions thallium au travers des canaux hKv1.5. La mesure est réalisée 30 sec après l'injection de la solution dépolarisante. L'application de 10 µM DPO (Tocris, bloqueur des canaux Kv1.5) permet de normaliser la fluorescence.

**Tableau 1**

| Exemples | % inhibition à 10 µM |
|---|---|
| BMS394136 | 99,6 |
| 1 | 100 |
| 2 | 100 |
| 3 | 43,3 |
| 4 | 54,9 |
| 5 | 93,6 |
| 6 | 94,2 |
| 7 | 54,9 |
| 8 | 88 |
| 9 | 60,1 |

| | |
|---|---|
| * BMS394136 est un bloqueur du canal Kv1.5 en développement chez Bristol-Myers Squibb (Abstract, D. Xing et al. Circulation 2009, 120 (18S3): 2515). | |

Les résultats obtenus montrent que les composés de formule générale (I) bloquent le canal Kv1.5

Les composés de formule générale (I) peuvent être utilisés en tant que bloqueurs du canal Kv1.5.

### D) ABREVIATIONS

- CCM: Chromatographie sur Couche Mince
- DMF: Diméthylformamide
- DMSO: Diméthylsulfoxyde
- DPO: (2-isopropyl-5-méthyl-cyclohexyl) diphénylphosphine oxide
- HPLC: Chromatographie liquide haute performance
- Rf: Rapport frontal
- RMN: Résonance magnétique nucléaire
- THF: Tétrahydrofurane

## Revendications

1. Composés de formule générale I : 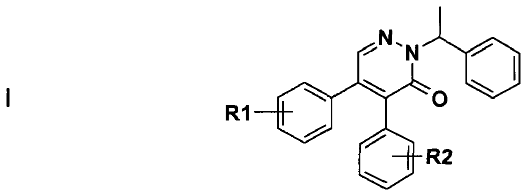 dans laquelle
R₁ et R₂ représentent de manière simultanée ou indépendamment un ou plusieurs groupements choisis parmi : halogène tel que F, Br, Cl, alkyle linéaire ou branché en C₁-C₄, hydroxy, alkoxy linéaire ou branché en C₁-C₄, nitrile ou arylsulfonamido dont l'aryl est éventuellement substitué par un groupement alkyle linéaire ou branché en C₁-C₄,
ainsi que les différents énantiomères et leurs mélanges en toutes proportions, et leurs sels pharmaceutiquement acceptables.

2. Composés de formule générale I selon la revendication 1) **caractérisés en ce que** 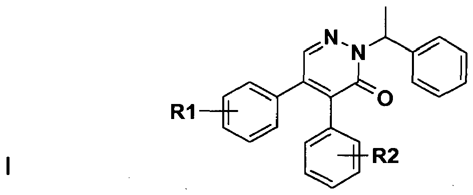
R₁ représente un groupement hydroxy, méthoxy ou cyano ;
R₂ représente plusieurs groupements choisis parmi : halogène tel que F, Br, Cl, alkyle linéaire ou branché en C₁-C₄, hydroxy, alkoxy linéaire ou branché en C₁-C₄, nitrile ;
ainsi que les différents énantiomères et leurs mélanges en toutes proportions, et leurs sels pharmaceutiquement acceptables.

3. Composés de formule générale I selon l'une des revendications 1) à 2) **caractérisés en ce que** : 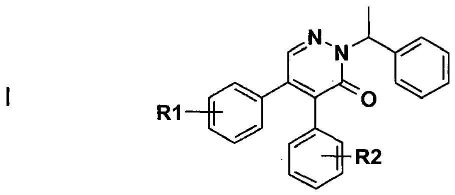
R₁ représente un groupement hydroxy ;
R₂ représente plusieurs groupements choisis parmi : halogène tel que F, Cl, alkyle linéaire ou branché en C₁-C₄, hydroxy, alkoxy linéaire ou branché en C₁-C₄, nitrile ;
ainsi que les différents énantiomères et leurs mélanges en toutes proportions, et leurs sels pharmaceutiquement acceptables.

4. Composés de formule générale I selon l'une des revendications 1) à 3)
**caractérisés en ce que** 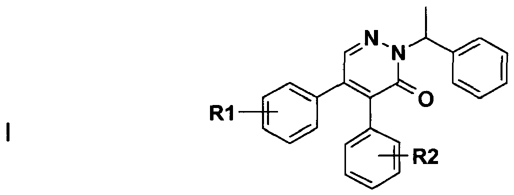
R₁ représente un groupement hydroxy situé en position para (position 4) sur le phényl qu'il substitue ;
R₂ représente plusieurs groupements choisis parmi : Cl, méthyle, hydroxy, méthoxy, nitrile ;
ainsi que les différents énantiomères et leurs mélanges en toutes proportions, et leurs sels pharmaceutiquement acceptables.

5. Composés de formule générale I selon l'une des revendications revendication 1) à 4) **caractérisés en ce qu'**ils sont choisis parmi :
1. 4,5-Bis-(4-hydroxy-phényl)-2-(1-phényl-éthyl)-2H-pyridazin-3-one
2. 4,5-Bis-(4-hydroxy-phényl)-2-((S)-1-phényl-éthyl)-2H-pyridazin-3-one
3. 4,5-Bis-(4-hydroxy-phényl)-2-((R)-1-phényl-éthyl)-2H-pyridazin-3-one
4. 2,2'-(6-oxo-1-(1-phényléthyl)-1,6-dihydropyridazine-4,5-diyl)dibenzonitrile
5. 3,3'-(6-oxo-1-(1-phényléthyl)-1,6-dihydropyridazine-4,5-diyl)dibenzonitrile
6. 4,5-Bis-(4-méthoxy-phényl)-2-(1-phényl-éthyl)-2H-pyridazin-3-one
7. N,N'-(3,3'-(6-oxo-1-(1-phényléthyl)-1,6-dihydropyridazine-4,5-diyl)bis(3,1-pheylee))bis(4-méthylbenzenesulfonamide)
8. 3-(5-(4-méthoxyphényl)-6-oxo-1-(1-phényléthyl)-1,6-dihydropyridazin-4-yl)-benzonitrile
9. 2-[5-(4-Méthoxy-phényl)-6-oxo-1-(1-phényl-éthyl)-1,6-dihydro-pyridazin-4-yl]-benzon itrile
10. N-{3-[5-(3,4-Diméthyl-phényl)-6-oxo-1-(1-phényl-éthyl)-1,6-dihydro-pyridazin-4-yl]-phényl}-4-méthyl-benzenesulfonamide
11.4,5-Bis-(3,4-dichloro-phényl)-2-(1-phényl-éthyl)-2H-pyridazin-3-one

6. Procédé de préparation des composés chimiques de formule générale I selon l'une des revendications 1) à 5) **caractérisé en ce que** l'on condense une dibromo ou dichloro pyridazinone de formule générale Il pour lequel X représente soit un atome chlore ou un atome de brome, 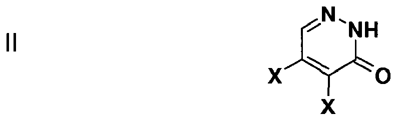 avec un dérivé de formule générale III, 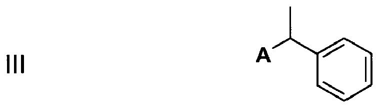 pour lequel :
- lorsque A représente un atome d'halogène tel qu'un atome chlore ou un atome de brome, on utilise une base telle que Cs₂CO₃ dans un solvant tel que le diméthylformamide.
- lorsque A représente OH, on utilise des conditions de couplage de Mitsunobu telles qu'en présence de diéthylazodicarboxylate d'éthyle et de triphénylphosphine dans un solvant tel que le THF ;
l'intermédiaire IV obtenu 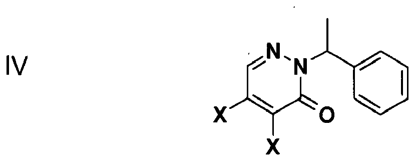 est alors couplé (étape 1) avec un dérivé du bore V 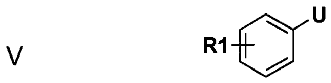 pour lequel R1 est tel que défini dans la formule générale I et U représente B(OH)₂ ou 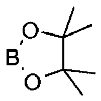 dans un mélange de solvants tel que toluène/éthanol ou eau/acétonitrile ou dioxane/eau en présence d'une base telle que le carbonate de sodium ou de potassium et d'un catalyseur tel que le tétrakis(triphénylphosphine)palladium ou PdCl₂/2PPh₃ ;
**en ce que** l'on obtient majoritairement la formation du composé VI et minoritairement à la formation du composé VII ;
l'intermédiaire VI étant alors remis en réaction (étape 2) :
- soit avec le dérivé du bore V dans les conditions de couplage décrites précédemment pour conduire à la formation du composé VII 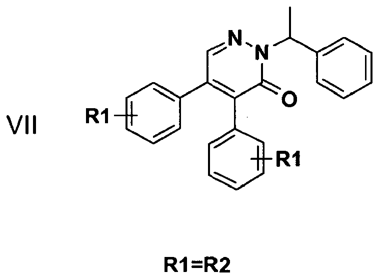
- soit avec le dérivé du bore VIII 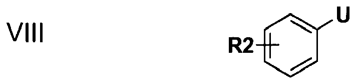 pour lequel R₂ est tel que défini dans la formule générale I et U est tel que défini précédemment dans les conditions de couplage décrites précédemment pour l'étape 1 pour conduire à la formation du composé IX .

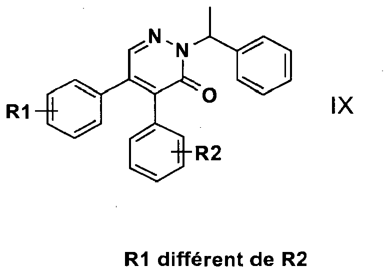

7. Composés de formule générale I tels que définis selon l'une des revendications 1) à 5) pour leur utilisation en tant que médicament.

8. Composés selon la revendication 7 pour leur utilisation dans le traitement et/ou à la prévention de la fibrillation atriale, des troubles du rythme cardiaque des oreillettes et/ou des ventricules, et des pathologies dans lesquelles le cycle cellulaire et/ou la prolifération cellulaire et/ou la régénération sont altérés tels que le cancer ou l'inflammation chronique.

9. Composition pharmaceutique comprenant un composé de formule générale I selon l'une quelconque des revendications 1) à 5) en association avec au moins un excipient pharmaceutiquement acceptable.

## Patentansprüche

1. Verbindungen der allgemeinen Formel I: 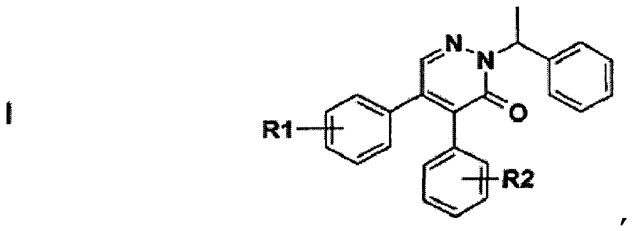 in der
R₁ und R₂ gleichzeitig oder unabhängig für eine oder mehrere Gruppen stehen, die ausgewählt sind aus: Halogen wie F, Br, Cl, linearem oder verzweigtem C₁-C₄-Alkyl, Hydroxy, linearem oder verzweigtem C₁-C₄-Alkoxy, Nitril oder Arylsulfonamido, dessen Aryl gegebenenfalls durch eine lineare oder verzweigte C₁-C₄-Alkylgruppe substituiert ist, sowie die verschiedenen Enantiomere und ihre Mischungen in jeglichen Anteilen und ihre pharmazeutisch unbedenklichen Salze.

2. Verbindungen der allgemeinen Formel I nach Anspruch 1, **dadurch gekennzeichnet, dass**: 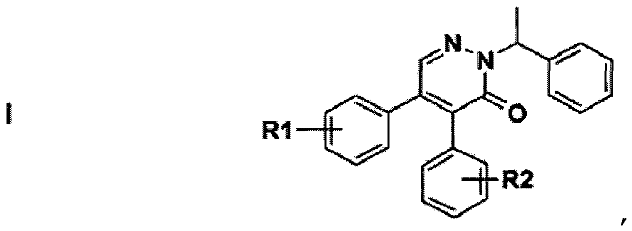
R₁ für eine Hydroxy-, Methoxy- oder Cyanogruppe steht;
R₂ für mehrere Gruppen steht, die ausgewählt sind aus: Halogen wie F, Br, Cl, linearem oder verzweigtem C₁-C₄-Alkyl, Hydroxy, linearem oder verzweigtem C₁-C₄-Alkoxy, Nitril;
sowie die verschiedenen Enantiomere und ihre Mischungen in jeglichen Anteilen und ihre pharmazeutisch unbedenklichen Salze.

3. Verbindungen der allgemeinen Formel I nach einem der Ansprüche 1 bis 2, **dadurch gekennzeichnet, dass**: 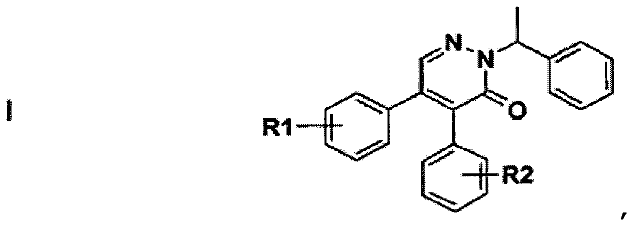
R₁ für eine Hydroxygruppe steht;
R₂ für mehrere Gruppen steht, die ausgewählt sind aus: Halogen wie F, Br, Cl, linearem oder verzweigtem C₁-C₄-Alkyl, Hydroxy, linearem oder verzweigtem C₁-C₄-Alkoxy, Nitril;
sowie die verschiedenen Enantiomere und ihre Mischungen in jeglichen Anteilen und ihre pharmazeutisch unbedenklichen Salze.

4. Verbindungen der allgemeinen Formel I nach einem der Ansprüche 1 bis 3, **dadurch gekennzeichnet, dass**: 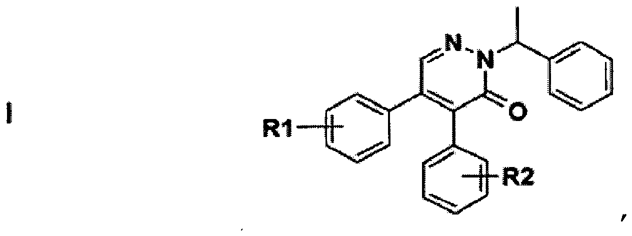
R₁ für eine Hydroxygruppe steht, die sich an dem Phenyl, das sie substituiert, in para-Stellung (4-Stellung) befindet;
R₂ für mehrere Gruppen steht, die ausgewählt sind aus: Cl, Methyl, Hydroxy, Methoxy, Nitril;
sowie die verschiedenen Enantiomere und ihre Mischungen in jeglichen Anteilen und ihre pharmazeutisch unbedenklichen Salze.

5. Verbindungen der allgemeinen Formel I nach einem der Ansprüche 1 bis 4, **dadurch gekennzeichnet, dass** sie ausgewählt sind aus:
1. 4,5-Bis-(4-hydroxyphenyl)-2-(1-phenylethyl)-2H-pyridazin-3-on
2. 4,5-Bis-(4-hydroxyphenyl)-2-((S)-1-phenylethyl)-2H-pyridazin-3-on
3. 4,5-Bis-(4-hydroxyphenyl)-2-((R)-1-phenylethyl)-2H-pyridazin-3-on
4. 2,2'-(6-Oxo-1-(1-phenylethyl)-1,6-dihydropyridazin-4,5-diyl)dibenzonitril
5. 3,3'-(6-Oxo-1-(1-phenylethyl)-1,6-dihydropyridazin-4,5-diyl)dibenzonitril
6. 4,5-Bis-(4-methoxyphenyl)-2-(1-phenylethyl)-2H-pyridazin-3-on
7. N,N'-(3,3'-(6-Oxo-1-(1-phenylethyl)-1,6-dihydropyridazin-4,5-diyl)bis-(3,1-phenylen))bis(4-methylbenzolsulfonamid)
8. 3-(5-(4-Methoxyphenyl)-6-oxo-1-(1-phenylethyl)-1,6-dihydropyridazin-4-yl)benzonitril
9. 2-[5-(4-Methoxyphenyl)-6-oxo-1-(1-phenylethyl)-1,6-dihydropyridazin-4-yl]benzonitril
10. N-{3-[5-(3,4-Dimethylphenyl)-6-oxo-1-(1-phenylethyl)-1,6-dihydropyridazin-4-yl]-phenyl}-4-methylbenzolsulfonamid
11. 4,5-Bis-(3,4-dichlorphenyl)-2-(1-phenylethyl)-2H-pyridazin-3-on.

6. Verfahren zur Herstellung von chemischen Verbindungen der allgemeinen Formel I nach einem der Ansprüche 1 bis 5, **dadurch gekennzeichnet, dass** ein Dibrom- oder Dichlorpyridazinon der allgemeinen Formel II kondensiert wird, wobei X entweder für ein Chloratom oder ein Bromatom steht: 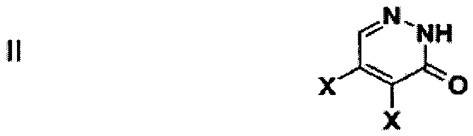 mit einem Derivat der allgemeinen Formel III: 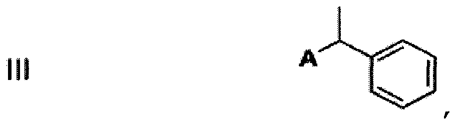 wobei:
- wenn A für ein Halogenatom wie ein Chloratom oder ein Bromatom steht, eine Base wie Cs₂CO₃ in einem Lösungsmittel wie Dimethylformamid verwendet wird,
- wenn A für OH steht, Mitsunobu-Kupplungsbedingungen wie in Gegenwart von Diethylazodicarbonsäureethylester und Triphenylphosphin in einem Lösungsmittel wie THF verwendet werden;
die erhaltene Zwischenverbindung IV: 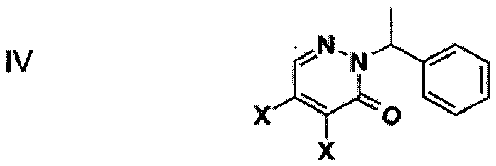 dann mit einem Borderivat V gekuppelt wird (Schritt 1): 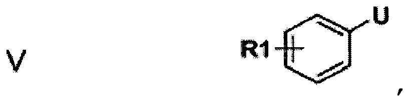 in dem R1 wie in der allgemeinen Formel I definiert ist und
U für B(OH)₂ oder 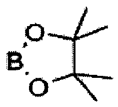 steht,
in einer Lösungsmittelmischung wie Toluol/Ethanol oder Wasser/Acetonitril in Gegenwart einer Base wie Natrium- oder Kaliumcarbonat und eines Katalysators wie Tetrakis(triphenylphosphin)palladium oder PdCl₂/2PPh₃;
dass zum großen Teil die Bildung der Verbindung VI und zum kleinen Teil die Bildung der Verbindung VII erzielt wird; die Zwischenverbindung VI dann in Reaktion mit folgendem gebracht wird (Schritt 2):
- entweder mit dem Borderivat V unter den oben beschriebenen Kupplungsbedingungen, um zur Bildung der Verbindung VII zu führen: 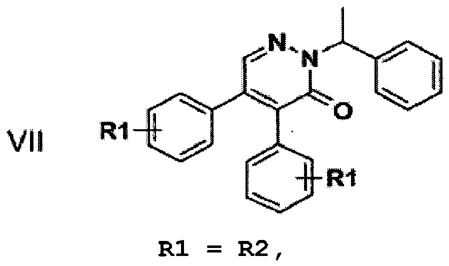
- oder mit dem Borderivat VIII: 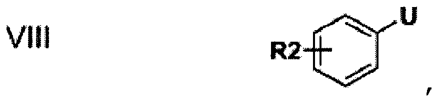
in dem R₂ wie in der allgemeinen Formel I definiert ist und U wie oben definiert ist, unter den oben beschriebenen Kupplungsbedingungen, um zur Bildung der Verbindung IX zu führen:

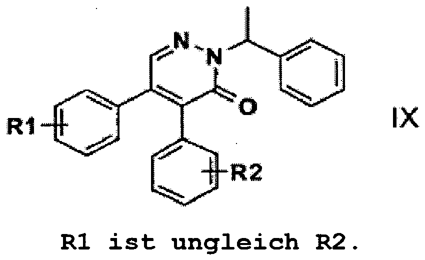

7. Verbindungen der allgemeinen Formel I, wie die in den Ansprüchen 1 bis 5 definierten, zu deren Verwendung als Arzneimittel.

8. Verbindungen nach Anspruch 7 zu deren Verwendung bei der Behandlung und/oder Verhinderung von Vorhofflimmern, Herzrhythmusstörungen der Herzohren und/oder der Herzkammern und Pathologien, in denen der Zellzyklus und/oder die Zellproliferation und/oder die Regeneration beeinträchtigt sind, wie Krebs oder chronische Entzündung.

9. Pharmazeutische Zusammensetzung, die eine Verbindung der allgemeinen Formel I nach einem der Ansprüche 1 bis 5 in Verbindung mit mindestens einem pharmazeutisch unbedenklichen Hilfsstoff umfasst.

## Claims

1. Compounds of general formula I: 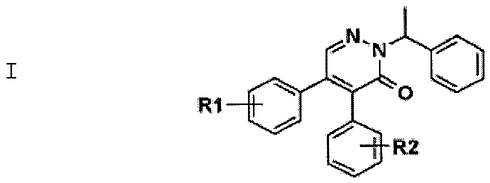 wherein
R₁ and R₂ simultaneously or independently represent one or several groups chosen from: halogen such as F, Br, Cl, linear or branched C₁-C₄ alkyl, hydroxy, linear or branched C₁-C₄ alkoxy, nitrile or arylsulfonamido the aryl of which is optionally substituted by a linear or branched C₁-C₄ alkyl group, as well as the different enantiomers and their mixtures in all proportions, and their pharmaceutically acceptable salts.

2. Compounds of general formula I according to claim 1) **characterised in that** 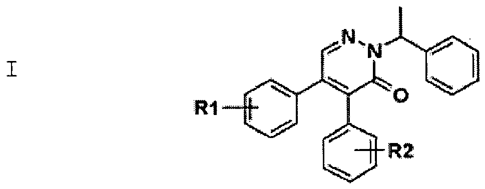
R₁ represents a hydroxy, methoxy or cyano group;
R₂ represents several groups chosen from: halogen such as F, Br, Cl, linear or branched C₁-C₄ alkyl, hydroxy, linear or branched C₁-C₄ alkoxy, nitrile;
as well as the different enantiomers and their mixtures in all proportions, and their pharmaceutically acceptable salts.

3. Compounds of general formula I according to any of claims 1) to 2) **characterised in that**: 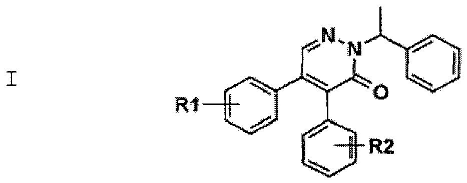
R₁ represents a hydroxy group;
R₂ represents several groups chosen from: halogen such as F, Cl, linear or branched C₁-C₄ alkyl, hydroxy, linear or branched C₁-C₄ alkoxy, nitrile;
as well as the different enantiomers and their mixtures in all proportions, and their pharmaceutically acceptable salts.

4. Compounds of general formula I according to any of claims 1) to 3) **characterised in that**: 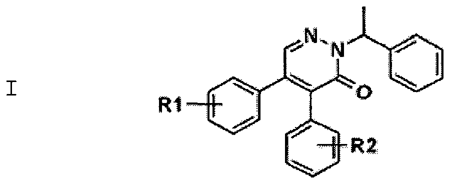
R₁ represents a hydroxy group located in para position (position 4) on the phenyl which it substitutes;
R₂ represents several groups chosen from: Cl, methyl, hydroxy, methoxy, nitrile;
as well as the different enantiomers and their mixtures in all proportions and their pharmaceutically acceptable salts.

5. Compounds of general formula I according to any of claims 1) to 4) **characterised in that** they are chosen from:
1. 4,5-Bis-(4-hydroxy-phenyl)-2-(1-phenyl-ethyl)-2H-pyridazin-3-one
2. 4,5-Bis-(4-hydroxy-phenyl)-2-((S)-1-phenyl-ethyl)-2H-pyridazin-3-one
3. 4,5-Bis-(4-hydroxy-phenyl)-2-((R)-1-phenyl-ethyl)-2H-pyridazin-3-one
4. 2,2'-(6-oxo-1-(1-phenylethyl)-1,6-dihydropyridazine-4,5-diyl)dibenzonitrile
5. 3,3'-(6-oxo-1-(1-phenylethyl)-1,6-dihydropyridazine-4,5-diyl)dibenzonitrile
6. 4,5-Bis-(4-methoxy-phenyl)-2-(1-phenyl-ethyl)-2H-pyridazin-3-one
7. N,N'-(3,3'-(6-oxo-1-(1-phenylethyl)-1,6-dihydropyridazine-4,5-diyl)bis(3,1-phenylene))bis(4-methylbenzenesulfonamide)
8. 3-(5-(4-methoxyphenyl)-6-oxo-1-(1-phenylethyl)-1,6-dihydropyridazin-4-yl)-benzonitrile
9. 2-[5-(4-Methoxy-phenyl)-6-oxo-1-(1-phenyl-ethyl)-1,6-dihydro-pyridazin-4-yl]-benzonitrile
10. N-{3-[5-(3,4-Dimethyl-phenyl)-6-oxo-1-(1-phenyl-ethyl)-1,6-dihydro-pyridazin-4-yl]-phenyl}-4-methyl-benzenesulfonamide
11. 4,5-Bis-(3,4-dichloro-phenyl)-2-(1-phenyl-ethyl)-2H-pyridazin-3-one

6. Process for preparation of the chemical compounds of general formula I according to any of claims 1) to 5) **characterised in that** a dibromo or dichloro pyridazinone of general formula II is condensed for which X represents either a chlorine atom or a bromine atom, 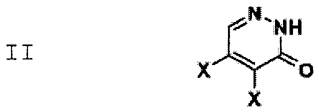 with a derivative of general formula III, 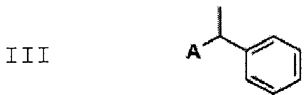 for which:
- when A represents a halogen atom such as a chlorine atom or a bromine atom, a base such as Cs₂CO₃ is used in a solvent such as dimethylformamide.
- when A represents OH, Mitsunobu coupling conditions are used such as in the presence of ethyl diethylazodicarboxylate and triphenylphosphine in a solvent such as THF;
the obtained intermediate IV 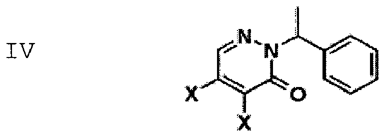 is subsequently coupled (step 1) with a boron derivative V 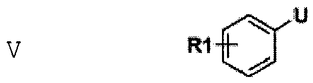 for which R1 is as defined in the general formula I and U represents
B(OH)₂ or 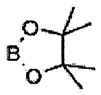 in a mixture of solvents such as toluene/ethanol or water/acetonitrile or dioxane/water in the presence of a base such as sodium or potassium carbonate and a catalyst such as tetrakis(triphenylphosphine)palladium or PdCl₂/2PPh₃;
**in that** one mainly obtains formation of compound VI and minimally obtains formation of compound VII;
intermediate VI being subsequently reacted again (step 2):
- either with the boron derivative V in the coupling conditions previously described in order to yield compound VII 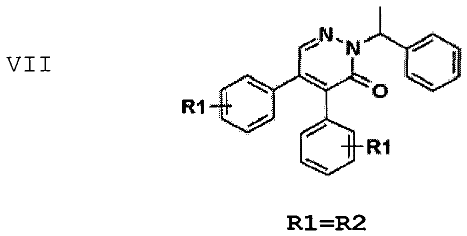
- or with the boron derivative VIII 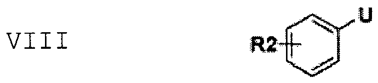
for which R₂ is as defined in general formula I and U is as defined above in the coupling conditions previously described for step 1 in order to yield compound IX.

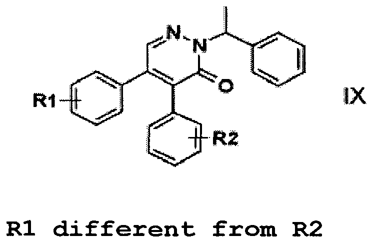

7. Compounds of general formula I as defined according to any of claims 1) to 5) for use thereof as a medicine.

8. Compounds according to claim 7 for use thereof in the treatment and/or prevention of atrial fibrillation, auricular and/or ventricular cardiac arrhythmias and of diseases in which the cell cycle and/or cell proliferation and/or regeneration are impaired such as cancer or chronic inflammation.

9. Pharmaceutical composition comprising a compound of general formula I according to any of claims 1) to 5) in combination with at least one pharmaceutically acceptable excipient.
